(19) Europäisches Patentamt
European Patent Office
Office européen des brevets

(11) Veröffentlichungsnummer: **0 186 028**
**B1**

(12) **EUROPÄISCHE PATENTSCHRIFT**

(45) Veröffentlichungstag der Patentschrift: **01.08.90**

(21) Anmeldenummer: **85115640.6**

(22) Anmeldetag: **09.12.85**

(51) Int. Cl.$^5$: **C 07 D 211/84,**
C 07 D 401/04,
A 61 K 31/445, A 61 K 31/505

# (54) Optisch aktive Nitrodihydropyridine, Verfahren zur Herstellung und ihre Verwendung in Arzneimitteln.

(30) Priorität: **22.12.84 DE 3447169**

(43) Veröffentlichungstag der Anmeldung:
**02.07.86 Patentblatt 86/27**

(45) Bekanntmachung des Hinweises auf die Patenterteilung:
**01.08.90 Patentblatt 90/31**

(84) Benannte Vertragsstaaten:
**AT BE CH DE FR GB IT LI NL SE**

(56) Entgegenhaltungen:
**EP-A-0 002 208**
**EP-A-0 026 317**
**EP-A-0 030 343**
**EP-A-0 039 863**
**EP-A-0 071 819**
**EP-A-0 088 903**
**DE-A-3 320 616**

**Chem. Pharm. Bull. 28, 2809 (1980)**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Franckowiak, Gerhard, Dr.**
**Henselweg 10**
**D-5600 Wuppertal 1 (DE)**
Erfinder: **Grosser, Rolf, Dr.**
**Gellertstrasse 9**
**D-5090 Leverkusen (DE)**
Erfinder: **Thomas, Günter, Dr.**
**Henselweg 5**
**D-5600 Wuppertal 1 (DE)**
Erfinder: **Schramm, Mathias, Dr.**
**Paffrather Strasse 38**
**D-5000 Köln 80 (DE)**
Erfinder: **Gross, Rainer, Dr.**
**Platzhofstrasse 23**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 186 028 B1

**Beschreibung**

Die vorliegende Erfindung betrifft optisch aktive 5-Nitro-dihydropyridinderivate, Verfahren zu ihrer Herstellung und ihre Verwendung in Arzneimitteln insbesondere in kreislaufwirksamen Arzneimitteln.

Es sind bereits optisch aktive 1,4-Dihydropyridine mit biologischen Wirkungen und Methoden zu ihrer Herstellung bekannt. In EP—A—0 039 836, EP—A—0 026 317, EP—A—0 088 903 und DE—A—3 320 616 und auch in der Publikation Chem. Pharm. Bull., 28, 2809—2812 (1980) werden Dihydropyridinderivate teilweise als optische Antipoden beschrieben, die sich in ihrer chemischen Struktur eindeutig von den 5-Nitro-dihydropyridin-Verbindungen der vorliegenden Erfindung unterscheiden. Bei diesen Publikationen und Anmeldungen handelt es sich im wesentlichen um klassische Dihydropyridinstrukturen mit Esterfunktionen in 3- und 5-Position des Dihydropyridinringes. In EP—A—0 071 819 und EP—A—0 002 208 werden auch bereits Verbindungen beschrieben, die am Dihydropyridinring einen Nitrosubstituenten in 3- bzw. 5-Position tragen. Optische Antipoden oder detaillierte Methoden zu ihrer Herstellung werden dort aber nicht genannt. Auch enthalten diese Vorpublikationen keinen Hinweis auf die überraschenden Eigenschaften der erfindungsgemäß ausgewählten Verbindungen.

Überraschenderweise zeigen die erfindungsgemäß ausgewählten optisch aktiven Dihydropyridinderivate nicht nur quantitative Wirkungsunterschiede gegenüber dem Racemat bzw. untereinander sondern gravierende qualitative Unterschiede, für die der Stand der Technik keinen Hinweis gibt.

Die vorliegende Erfindung betrifft (+)- und (−)-Enantiomere von 5-Nitrodihydropyridinen der allgemeinen Formel I

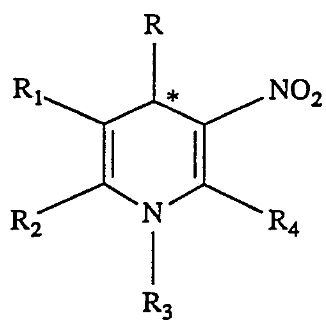

(I)

im welcher

R für Furyl, Thienyl, Pyridyl, Pyrimidyl, oder für Phenyl steht, der gegebenenfalls ein- oder zweimal, gleich oder unterschiedlich substituiert ist durch Fluor, Chlor, Nitro, Cyano oder durch gegebenenfalls durch ein oder mehrere Fluor substituiertes Alkyl oder Alkoxy (jeweils 4 C-Atome), oder durch die Gruppe

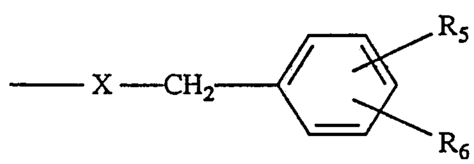

, wobei

X für Sauerstoff oder Schwefel steht, und

$R_5$ und $R_6$ gleich oder verschieden sind und für Wasserstoff, Nitro, Cyano oder für Alkyl, Alkyloxy, Alkylthio, Halogenalkyl, Halogenalkoxy (jeweils bis 2 C-Atome) steht und Halogen bevorzugt für ein oder mehrere Fluor steht,

$R_1$ für Wasserstoff oder für die Gruppe $CO_2R_8$ steht, wobei

$R_8$ für Wasserstoff oder für geradkettiges oder verzweigtes Alkyl (bis 6 C-Atome steht, das durch ein Sauerstoff- und/oder Schwefelatom in der Kette unterbrochen sein kann und das gegebenenfalls substituiert ist durch Phenyl, Nitro, ein oder mehrere Fluor, Chlor, Cyano, Benzyl-methylamino oder Pyridyl,

$R_2$, $R_4$ für Methyl oder Ethyl steht, das gegebenenfalls substituiert ist durch Hydroxy, ein oder mehrere Fluor, Phenyl oder Alkoxycarbonyl (bis 2 C-Atome),

$R_3$ für Wasserstoff oder für Methyl oder Ethyl steht, sowie deren Salze.

Die erfindungsgemäßen Stoffe können in Form ihrer Salze vorliegen. Dies sind im allgemeinen Salze mit anorganischen oder organischen Säuren. Bevorzugt sind die physiologisch unbedenklichen Salze der erfindungsgemäßen Stoffe mit organischen oder anorganischen Säuren. Als Beispiele seien genannt Hydrochloride, Hydrobromide, Hydrogensulfate, Sulfate, Hydrogenphosphate, Acetate, Maleate, Fumarate, Citrate, Tartrate oder Benzoate.

Ganz besonders bevorzugt sind die (−)-Enantiomeren der allgemeinen Formel (I).

Die erfindungsgemäßen (+)- bzw. (−)-Enantiomeren der allgemeinen Formel (I) werden hergestellt, indem man die optisch aktiven Aminocrotonsäureester der allgemeinen Formel (II)

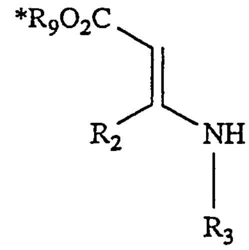

(II)

in welcher

$R_2$, $R_3$ die oben angegebene Bedeutung haben und

$*R_9$ für einen chiral einheitlichen 2-Methoxy-2-phenyl-ethylrest steht,

mit Ylidenverbindungen der allgemeinen Formel III

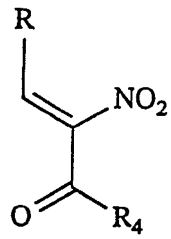

(III)

gegebenenfalls in Anwesenheit von Wasser oder inerten organischen Lösungsmitteln umsetzt, dann die hierbei, wegen der beiden möglichen verschiedenen Konfigurationen am $C_4$-Atom des Dihydropyridinringes, anfallenden Diastereomeren nach üblichen Methoden trennt, und die so erhaltenen 1,4-Dihydropyridine mit chiraler Estergruppierung der allgemeinen Formel IV

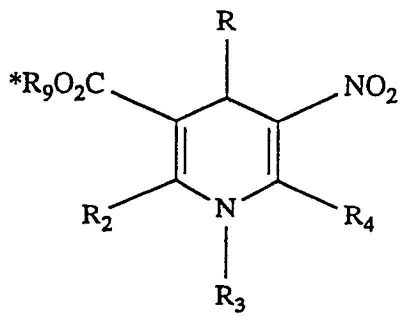

(IV)

in welcher

R, $R_1$, $R_2$, $R_3$, $R_4$ und $*R_9$ die oben angegebene Bedeutung haben, nach üblichen Methoden entweder umestert, indem man den chiralen Rest $*R_9$ gegen einen achiralen Rest $R_8$ ($\neq$ Wasserstoff) austauscht, oder hydrolysiert zu Verbindungen mit $R_8$ = Wasserstoff, und diese anschließend gegebenenfalls wieder verestert oder decarboxyliert zu Verbindungen mit $R^1$ = H, wobei jeweils (+)- bzw. (−)-Enantiomere der allgemeinen Formel I erhalten werden.

Die Ausgangsstoffe der Formel (II) und (IIII) sind bekannt oder können nach bekannten Methoden hergestellt werden (vgl. A. Dornow, W. Sassenberg, Liebigs Ann. Chem. *602*, 14 (1957); S. A. Glickmann, A. C. Cope, J. Am. Chem. Soc., *67*, 1017 (1945)).

Die Stoffe der Formel (IV) sind neu und können in der angegebenen Weise hergestellt werden.

Als Verdünnungsmittel kommen Wasser oder alle inerten organischen Lösungsmittel in Frage. Hierzu gehören bevorzugt Alkohole, wie Methanol, Ethanol, Propanol, Isopropanol, Ether wie Diethylether, Dioxan, Tetrahydrofuran oder Glykolmonoethylether, Eisessig, Pyridin, Dimethylformamid, Acetonitril, Dimethylsulfoxid oder Hexamethylphosphorsäuretriamid.

Die Reaktionstemperaturen können in einem größeren Bereich variiert werden. Im allgemeinen arbeitet man in einem Bereich von 10°C bis 200°C, bevorzugt von 20°C bis 150°C.

Die Umsetzung kann bei Normaldruck, aber auch bei erhöhtem Druck durchgeführt werden. Im allgemeinen arbeitet man bei Normaldruck.

Die gemäß den oben angegebenen Verfahren entstehenden Verbindungen der Formel (IV) unterschieden sich als Diasteromere in ihren physikalischen und chemischen Eigenschaften und können daher mit Hilfe bekannter Methoden voneinander getrennt werden. Als Trennmethoden seien vorzugsweise genannt: Umkristallisation aus inerten Lösungsmitteln, Dünnschicht-, Säulen- oder Hochdruckflüssigkeitschromatographie.

Die erforderliche Hydrolyse oder Umesterung der chiral einheitlichen Verbindungen IV erfolgt vorzugsweise über alkalische Hydrolyse oder Alkholyse, gegebenenfalls in Gegenwart eines inerten Lösungsmittels unter Verwendung von $R_8O^-$ als Reagenz, wobei $R_8$ die oben angegebene Bedeutung hat.

Als Lösungsmittel für diese Hydrolyse oder Umesterung kommen Wasser oder alle interten organischen Lösungsmittel oder Gemische derselben in Frage. Hierzu gehören vorzugsweise Alkohole wie Methanol, Ethanol, Propanol oder Isopropanol, Ether wie Dioxan, Tetrahydrofuran, Glykolmonoethylether, Glykoldimethylether oder Dimethylformamid, Dimethylsulfoxid, Acetonitril, Pyridin oder Hexamethylphosphorsäuretriamid.

Die Reaktionstemperaturen können in einem größeren Bereich variiert werden. Im allgemeinen arbeitet man zwischen 20 und 150°C, vorzugsweise bei ca. 50 bis 100°C.

Die Hydrolyse bzw. Umesterung kann bei Normaldruck, aber auch bei erhöhtem Druck durchgeführt werden. Im allgemeinen arbeitet man unter Normaldruck.

Als Alkoholyse- bzw. Hydrolysereagentien $R_8O^-$ kommen die üblichen Basen in Betracht. Bevorzugt werden Alkali- oder Erdalkalihydroxide wie zum Beispiel Natrium-, Kalium-, Calcium- oder Bariumhydroxid, oder Alkalialkoholate wie zum Beispiel Natrium- oder Kaliummethanolat oder Natrium- oder Kaliumethanolat eingesetzt. Ebenso ist es möglich, eine Mischung der Basen zu verwenden. Die Basen werden bei der Durchführung der Hydrolyse bzw. Alkoholyse jeweils in molaren Mengen oder in geringem Überschuß eingesetzt.

Die Decarboxylierungs der Verbindungen mit $R_1$ = COOH zu Verbindungen mit $R_1$ = H erfolgt in üblicher Weise. Bevorzugt wird thermisch, gegebenenfalls in Abwesenheit eines säuren Katalysators, durch Erhitzen der entsprechenden Carbonsäure mit oder ohne Lösungsmittel decarboxyliert.

Als Lösungsmittel für die Decarboxylierung kommen Wasser oder inerte organische Lösungsmittel oder aber Mischungen in Frage. Hierzu gehören vorzusweise Wasser, Alkohole wie z.B. Methanol, Ethanol, Propanol, Glykol oder Diglykol, Ether wie Dioxan, Tetrahydrofuran, Glykolmonoethylether, Glykoldimethylether, Diethylenglykoldimethylether oder Dimethylformamid, Dimethylsulfoxid, Acetonitril, Eisessig, Hexamethylphosphorsäuretriamid, Toluol oder Xylol.

Die Reaktionstemperaturen für die Decarboxylierung können in einem größeren Bereich variiert werden. Im allgemeinen arbeitet man zwischen 40 und 200°C, vorzugsweise zwischen 70 und 150°C.

Die Decarboxylierung kann bei Normaldruck, bei erhöhtem Druck oder erniedrigtem Druck durchgeführt werden. Im allgemeinen arbeitet man unter Normaldruck. Als Katalysatoren können die üblichen anorganischen oder organischen Säuren verwendet werden. Hierzu gehören vorzugsweise Halogenwasserstoffsäuren wie HCl, HBr, Schwefelsäure, Phosphorsäure, oder organische Säuren, wie Essigsäure, Ameinsensäure, Toluolsulfonsäure oder Methansulfonsäure.

Die Reveresterung der erfindungsgemäßen Carbonsäuren ($R_8$ = H) erfolgt nach bekannten Methoden, gegebenenfalls über ein reaktives Säurederivat wie beispielsweise aktivierte Ester, Hydroxysuccinimidester, Säureimidazolide, gemischte Anhydride, oder Umsetzung mit Dicyclohexylcarbodiimid.

Hierfür kommen die üblichen organischen Lösungsmittel in Frage. Hierzu gehören bevorzugt Kohlenwasserstoffe wie Dichlormethan, Trichlormethan, Tetrachlormethan oder 1,2-Dichlorethan, Ether wie Diethylether, Dioxan, Tetrahydrofuran oder 1,2-Dimethoxyethan, aromatische Kohlenwasserstoffe wie Toluol oder Xylol, Acetonitril, Nitromethan, Dimethylformamid, Hexamethylphosphorsäuretriamid, Pyridin oder Essigester.

Die Reaktionstemperaturen können hierbei in einem größeren Bereich variiert werden. Im allgemeinen arbeitet man in einem Bereich von −70°C bis +60°C, bevorzugt von −50°C bis +40°C.

Die Reveresterung kann bei Normaldruck, aber auch bei erhöhtem Druck durchgeführt werden. Im allgemeinen arbeitet man bei Normaldruck.

Bei der Durchführung ist das Verhältnis der Reaktanden beliebig. Es hat sich jedoch als zweckmäßig erwiesen, den entsprechenden Alkohol bis zu einem 20 fachen, bevorzugt bis zu einem 10 fachen molaren Überschuß einzusetzen.

Die Enantiomeren der allgemeinen Formel (I) erhält man ebenfalls, wenn man die entsprechenden Racemate mit Hilfe geeigneter Trennmethoden, gegebenenfalls unter Verwendung optisch aktiver Materialien durch z.B. Dünnschicht-, Säulen- oder Hochdruckflössigskeitschromatographie in die (+)- bzw. (+)-Enantiomeren der allgemeinen Formel (I) trennt.

Es ist bekannt, daß racemische Nitrodihydropyridine eine kontraktionskraftverstärkende, am Herzmuskel positiv inotrope Wirkung besitzen. Es ist außerdem bekannt, daß sich die pharmakologische Wirksamkeit der isolierten Enantiomeren von der des Racemats unterscheiden.

So besitzt oft eines der Isomeren eine stärkere, das andere eine schwächere Wirkung als das Racemat.

Es war aber nicht vorhersehbar, daß die erfindungsgemäßen Enantiomeren völlig unterschiedlich wirken. Überraschenderweise besitzt jeweils ein Enantiomeres eine gefäßdilatierende, am Herzmuskel negativ inotrope Wirkung, während das andere Enantiomere gefäßkontrahierend, am Herzmuskel positiv inotrop wirkt.

Im einzelnen konnten für die gefäßdilatierenden Enantiomeren im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B dem Zentralnervensystem) manifestieren. Die Verbindungen eignen sich daher auch als Celebraltherapeutika.

4. Die Verbindungen senken den Blutdruck von normotonen und hypertonen Tieren und können somit als antihypertensive Mittel verwendet werden.

5. Verbindungen haben stark muskulär-spasmolytische Wirkungen, die an der glatten Muskulatur des Magens, Darmtraktes, des Urogenitaltraktes und des Respirationssystems deutlich werden.

Diese erfindungsgemäßen Enantiomeren eignen sich aufgrund dieser Eigenschaften besonders zur Prophylaxe und Therapie der akuten oder chronischen ischämischen Herzkrankheiten sowie zur Behandlung von cerebralen und peripheren Durchblutungsstörungen.

Die optischen Antipoden zu diesen Enantiomeren haben eine positive inotrope Wirkung und zeigen somit ein nicht vorhersehbares und wertvolles pharmakologisches Wirkspektrum. Sie können als Cardiotonika zu Verbesserung der Herzkontraktilität dienen. Darüber hinaus können sie als Antihypotonika, zur Senkung von Blutzucker, zur Abschwellung von Schleimhäuten und zur Beeinflussung des Salz- und Flüssigkeitshaushaltes eingesetzt werden.

Außerdem betrifft die Erfindung eine neue Wirkstoffkombination, enthaltend (−)-Enantiomere der Formel (I) (Komponente X) und (+)-Enantiomere der Formel (I) (Komponente Y).

Die Kombination zeigt bei geeigneten Verhältnissen der Komponenten X und Y ein völlig überraschendes Wirkprofil: Sie sind positiv inotrop und gefäß- insbesondere coronardilatierend.

Bezogen auf ein Gewichtsteil der Komponente X können 0,01—1000 Gewichtsteile, bevorzugt 0,1—50 Gewichtsteile der Komponente Y eingesetzt werden.

Die Kombinationen können hergestellt werden, indem man die Einzelkomponenten in diese auflösenden inerten Lösungsmittel auflöst und die Lösungen anschließend mischt.

Als inerte Lösungsmittel seien beispielshaft Alkohole wie Ethanol oder Glykole wie Polyethylenglykol oder Dimethylsulfonid genannt.

Die erfindungsgemäße Wirkstoffkombination kann zur Bekämpfung von Krankheiten, insbesondere von Kreislauferkrankungen, verwendet werden.

Die Herz- und Gefäßwirkungen der erfindungsgemäßen Enantiomeren sowie Kombination wurden an isoliert perfundierten Herzen des Meerschweinchens gefunden (modifiziert nach Opie, L., J. Physiol. *180* (1965) 529—541). Dazu werden die Herzen von 250 bis 350 g schweren Albino Meerschweinchen verwendet. Die Tiere werden mit einem Schlag auf den Kopf getötet, der Thorax geöffnet, in die freipräparierte Aorta eine Metallkanüle eingebunden und der linke Vorhof geöffnet. Das Herz wird mit den Lungen aus dem Thorax herausgetrennt und über die Aortenkanüle an die Perfusionsapparatur bei laufender Perfusion angeschlossen. Die Lungen werden an den Lungenwurzeln abgetrennt. Als Perfusionsmedium dient Krebs-Henseleit-Lösung (118,5 mmol/l NaCl, 4,75 mmol/l KCl, 1,19 mmol/l $KH_2PO_4$, 119 mmol/l $MgSO_4$, 25 mmol/l $NaHCO_3$, 0,013 mmol/l NaEDTA), deren $CaCl_2$ Konzentration je nach Bedarf variiert wird, in der Regel jedoch 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$ zur Aufrechterhaltung des pH-Wertes von 7,4) begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert.

De Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdruckes eine Coronardilatation, eine Steigerung der linksventrikulären Druckamplitude einen Anstieg der Herzokontraktilität an. Die erfindungsgemäßen Enantiomeren bzw. deren Kombinationen werden in geeigneten Verdünnungen in das Perfusionssystem kurz vor dem isolierten Herzen infundiert.

Tabelle 1 zeigt die Wirkung einiger Stereoisomer auf Kontraktilität und Koronarwiderstand isolierter Meerschweinchenherzen.

Isomeres 1: (+)-1,4-Dihydro-2,6-dimethyl-5-nitro-4-(2-trifluormethylphenyl)-pyridin-3-carbonsäuremethylester

Isomeres 2: (−)-1,4-Dihydro-2,6-dimethyl-5-nitro-4-(2-trifluormethylphenyl)-pyridin-3-carbonsäuremethylester

Isomeres 3: (+)-(2-Benzylthiophenyl)-1,4-dihydro-2,6-dimethyl-5-nitro-pyridin-3-carbonsäuremethylester

Isomeres 4: (−)-(2-Benzylthiophenyl)-1,4-dihydro-2,6-dimethyl-5-nitro-pyridin-3-carbonsäuremethylester

TABELLE 1

| Isomeres Nr. | Konzentration (g/mol) | Änderung KA (%) | Änderung PD (%) |
|---|---|---|---|
| 1 | $3 \cdot 10^{-8}$ | − 21 | − 12 |
| 1 | $3 \cdot 10^{-7}$ | − 76 | − 18 |
| 2 | $3 \cdot 10^{-9}$ | + 47 | + 8 |
| 2 | $3 \cdot 10^{-8}$ | + 86 | + 20 |
| 3 | $10^{-7}$ | 0 | − 15 |
| 3 | $10^{-6}$ | − 26 | − 31 |
| 4 | $10^{-10}$ | + 2 | + 22 |
| 4 | $10^{-9}$ | +106 | + 20 |

KA = Kontaktionsamplitude
PD = Perfusionsdruck

Tabelle 2 zeigt besipielhaft die kontraktilitätssteigernde und coronardilatierende Wirkung von Kombinationen am isoliert perfundierten Meerschweinchenherzen:

Kombination 1:
bestehend aus
(−)-1,4-Dihydro-2,6-dimethyl-5-nitro-4-(2-trifluormethylphenyl)-pyridin-3-carbonsäuremethylester
und
(+)-1,4-Dihydro-2,6-dimethyl-5-nitro-4-(2-trifluormethylphenyl)-pyridin-3-carbonsäuremethylester
im Verhältnis 1:10.

Kombination 2:
bestehend aus
(−)-1,4-Dihydro-2,6-dimethyl-5-nitro-4-(2-trifluormethylphenyl)-pyridin-3-carbonsäuremethylester
und
(+) 4-(2-Benzylthiophenyl)-1,4-dihydro-3-nitro-pyridin-3-carbonsäuremethylester
im Verhältnis 1:30.

| Kombination | Konzentrationen | Ka | PD |
|---|---|---|---|
| 1 | $10^{-9}/10^{-8}$ g/ml | + 29% | − 8% |
| | $10^{-8}/10^{-7}$ g/ml | + 48% | − 17% |
| 2 | $3 \times 10^{-9}/9 \times 10^{-8}$ g/ml | + 68% | − 21% |
| | $10^{-8}/3 \times 10^{-7}$ g/ml | + 75% | − 27% |

KA = Kontraktionsamplitude
PD = Perfusionsdruck

Sowohl die Einzelwirkstoffe, als auch die Wirkstoffkombination können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei sollen die therapeutisch wirksamen Verbindungen bzw. Kombination jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Diese Formulierungen werden beispielsweise hergestellt durch Verstrecken der Wirkstoffkombination bzw. Einzelwirkstoffe mit Lösungsmitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe wie z.B. natürliche Gesteinsmehle (z.B. Kaoline, Tonerden, Talkum, Kreide), synthetisches Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe bzw. Kombinationen außer mit den obengenannten Hilfsstoffen mit verschiedenen Geschmacksaufbesserern oder Farbstoffen versetzt werden.

Für den Fall der parenteralen Anwendung können Lösungen der Wirkstoffkombination bzw. Einzelwirkstoffe unter Verwendung geeigneter flüssiger Trägermaterialien eingesetzt werden.

## Herstellungsbeispiele

### Beispiel 1

a) (+)-1,4-Dihydro-2,6-dimethyl-5-nitro-4-(2-trifluormethylphenyl)-pyridin-3-carbonsäure-[(S)-2-methoxy-2-phenyl-ethyl]ester

2.35 g (10 mmol) (+)-(S)-3-Aminocrotonsäure-(2-methoxy-2-phenylethyl)ester werden mit 2.59 g (10 mmol) 2-Nitro-1-(2-trifluormethylphenyl)-buten-1-on-3 in 20 ml i-Propanol zum Rückfluß erhitzt. Aus der heißen Lösung kristallisiert nach Anreiben das Produkt aus, das dann aus heißem Isopropanol umkristallisiert und heiß abgesaugt wird.

Ausbeute: 2.1 g (44% der Theorie)
Schmelzpunkt: 209°C (Zers.)
Drehwert $[\alpha]_D^{20} = + 144.47°$ (Dioxan)

b) (+)-1,4-Dihydro-2,6-dimethyl-5-nitro-4-(2-trifluormethylphenyl)-pyridin-3-carbonsäuremethylester

1.19 g (2.5 mmol) (+)-1,4-Dihydro-2,6-dimethyl-5-nitro-4-(2-trifluormethylphenyl)-pyridin-3-carbonsäure-[(S)-2-methoxy-2-phenylethyl)ester werden in 100 ml Methanol mit 270 mg Natriummethylat 50 h auf 50°C erhitzt. Anschließend wird mit methanolischer HCl auf pH 3 eingestellt, eingedampft und der Rückstand mit Chloroform/Methanol (3%) auf Kieselgel chromatographiert.

Ausbeute: 480 mg (54% der Theorie)
Schmelzpunkt: 177°C
Drehwert $[\alpha]_D^{20} = + 46.8°$ (Dioxan)

### Beispiel 2

a) (−)-1,4-Dihydro-2,6-dimethyl-5-nitro-4-(2-trifluormethylphenyl)-pyridin-3-carbonsäure-[(R)-2-methoxy-2-phenylethyl]ester

2.35 g (10 mmol) (−)-(R)-3-Amino-crotonsäure-(2-methoxy-2-phenylethyl)ester werden mit 2.59 g (10 mmol) 2-Nitro-1-(2-trifluormethylphenyl)-buten-1-on-3 in 20 ml i-Propanol zum Rückfluß erhitzt. Aus der heißen Lösung kristallisiert nach Anreiben das Produkt aus, es wird aus der heißen Lösung isoliert.

Ausbeute: 2.3 g (48% der Theorie)
Schmelzpunkt: 208°C (Zers.)
Drehwert $[\alpha]_D^{20} = - 142.47°$ (Dioxan)

b) (−)-1,4-Dihydro-2,6-dimethyl-5-nitro-4-(2-trifluormethylphenyl)-pyridin-3-carbonsäuremethylester

1.19 g (2.5 mmol) (−)-1,4-Dihydro-2,6-dimethyl-5-nitro-4-(2-trifluormethylphenyl)-pyridin-3-carbonsäure-[(R)-2-methoxy-2-phenylethyl)ester werden 3 h in 100 ml Methanol mit 270 mg Natriummethylat auf 50°C erhitzt. Es wird mit methanolischer HCl auf pH 3 eingestellt, eingedampft und auf Kieselgel mit Chloroform + 3% Methanol chromatographiert.

Ausbeute: 510 mg (57% der Theorie)
Schmelzpunkt: 176°C
Drehwert $[\alpha]_D^{20} = - 56.7°$ (Dioxan)

Analog Beispiel 1 und 2 wurden hergestellt:

### Beispiel 3

(+)-4-(2-Benzyloxyphenyl)-1,4-dihydro-2,6-dimethyl-5-nitro-pyridin-3-carbonsäuremethylester

Drehwert $[\alpha]_D^{20} = + 83.5°$ (Dioxan)

Beispiel 4

(−)-4-(2-Benzyloxyphenyl)-1,4-dihydro-2,6-dimethyl-5-nitro-pyridin-3-carbonsäuremethylester

Drehwert $[\alpha]_D^{20} = -93.3°$ (Dioxan)

Beispiel 5

(+)-4-(2-Difluormethoxyphenyl)-1,4-dihydro-2,6-dimethyl-5-nitro-pyridin-3-carbonsäuremethylester

Drehwert $[\alpha]_D^{20} = +14.7°$

Beispiel 6

(−)-4-(2-Difluormethoxyphenyl)-1,4-dihydro-2,6-dimethyl-5-nitro-pyridin-3-carbonsäuremethylester

Drehwert $[\alpha]_D^{20} = -16.1°$

Beispiel 7

(+)-4-[2-(3-Nitro)-benzylthio-phenyl]-1,4-dihydro-2,6-dimethyl-5-nitro-pyridin-3-carbonsäure-methylester

Drehwert $[\alpha]_D^{20} = +35.2°$ (Dioxan)

Beispiel 8

(−)-4-[2-(3-Nitro)-benzylthio-phenyl]-1,4-dihydro-2,6-dimethyl-5-nitro-pyridin-3-carbonsäure-methylester

Drehwert $[\alpha]_D^{20} = -42.7°$ (Dioxan)

Beispiel 9

(+)-4-(2-Benzylthiophenyl]-1,4-dihydro-2,6-dimethyl-5-nitro-pyridin-3-carbonsäuremethylester

Drehwert $[\alpha]_D^{20} = +56.1°$ (Dioxan)

Beispiel 10

(−)-4-(2-Benzylthiophenyl]-1,4-dihydro-2,6-dimethyl-5-nitro-pyridin-3-carbonsäuremethylester

Drehwert $[\alpha]_D^{20} = -58.4°$ (Dioxan)

Beispiel 11

(+)-1,4-Dihydro-2,6-dimethyl-5-nitro-4-(5-pyrimidyl)-pyridin-3-carbonsäuremethylester

Drehwert $[\alpha]_{546}^{20} = +37.7°$ (Aceton)

Beispiel 12

(−)-1,4-Dihydro-2,6-dimethyl-5-nitro-4-(5-pyrimidyl)-pyridin-3-carbonsäuremethylester

Drehwert $[\alpha]_{546}^{20} = -39.1°$ (Aceton)

Beispiel 13

(−)-1,4-Dihydro-2,6-dimethyl-5-nitro-4-(2-trifluormethylphenyl)-pyridin-3-carbonsäure

1.19 g (2.5 mmol) (+)-1,4-Dihydro-2,6-dimethyl-5-nitro-4-(2-trifluormethylphenyl)-pyridin-3-carbonsäure-[(S)-2-methoxy-2-phenylethyl]ester werden mit 300 mg Natriumhydroxid in 100 ml Methanol 15 h auf 40°C erhitzt. Es wird anschließend eingedampft, der Rückstand in Chloroform aufgenommen und 2 mal mit 20 Wasser gewaschen. Die wäßr. Phase wird mit verd. HCl langsam auf pH 3 eingestellt, das Produkt kristallisiert aus und wird abgesaugt.

Ausbeute: 320 mg (37% der Theorie)
Schmelzpunkt: 218°C (Zers.)
Drehwert $[\alpha]_{546}^{20} = -10.6°$ (Aceton)

Beispiel 14

(+)-1,4-Dihydro-2,6-dimethyl-5-nitro-4-(2-trifluormethylphenyl)-pyridin-3-carbonsäure

1.19 g (2.5 mmol) (−)-1,4-Dihydro-2,6-dimethyl-5-nitro-4-(2-trifluormethylphenyl)-pyridin-3-carbonsäure-[(R)-2-methoxy-2-phenylethyl]ester werden mit 300 mg Natriumhydroxid in 100 ml Methanol 15 h auf 40°C erhitzt. Es wird anschließend eingedampft, der Rückstand in Chloroform aufgenommen und 2 mal mit 20 Wasser gewaschen. Die wäßr. Phase wird mit verd. HCl langsam auf pH 3 eingestellt, das Produkt kristallisiert aus und wird abgesaugt.

Ausbeute: 345 mg (40% der Theorie)
Schmelzpunkt: 217°C (Zers.)
Drehwert $[\alpha]_{546}^{20} = +11.5°$ (Aceton)

Beispiel 15

(−)-1,4-Dihydro-2,6-dimethyl-3-nitro-4-(2-trifluormethylphenyl)-pyridin

171 mg (0.5 mmol) (−)-1,4-Dihydro-2,6-dimethyl-5-nitro-4-(2-trifluormethylphenyl)-pyridin-3-carbonsäure werden in 10 ml Methanol mit 100 mg konz. Schwefelsäure 7 h zum Rückfluß erhitzt. Anschließend

wird mit 50 ml $H_2O$ verdünnt. Das ausgefallene Produkt wird abgesaugt und aus i-Propanol umkristallisiert.

Ausbeute: 122 mg (82% der Theorie)
Schmelzpunkt: 210°C
Drehwert $[\alpha]_D^{20} = -583°$ (Aceton)

Beispiel 16

(+)-1,4-Dihydro-2,6-dimethyl-3-nitro-4-(2-trifluormethylphenyl)-pyridin

171 mg (0.5 mmol) (+)-1,4-Dihydro-2,6-dimethyl-5-nitro-4-(2-trifluormethylphenyl)-pyridin-3-carbonsäure werden in 10 ml Methanol mit 100 mg konz. Schwefelsäure 7 h zum Rückfluß erhitzt. Anschließend wird mit 50 ml $H_2O$ verdünnt. Das ausgefallene Produkt wird abgesaugt und aus i-Propanol umkristallisiert.

Ausbeute: 114 mg (76% der Theorie)
Schmelzpunkt: 211°C
Drehwert $[\alpha]_D^{20} = -587°$ (Aceton)

Beispiel 17

(−)-1,4-Dihydro-2,6-dimethyl-5-nitro-4-(2-trifluormethylphenyl)-pyridin-3-carbonsäure-(3-brompropyl)ester

171 mg (0.5 mmol) (+)-1,4-Dihydro-2,6-dimethyl-5-nitro-4-(2-trifluormethylphenyl)-pyridin-3-carbonsäure werden in 15 ml Tetrahydrofuran Raumtemperatur mit 700 mg (5 mmol) 3-Brompropanol und 123 mg (0.6 mmol) Dicyclohexylcarbodiimid versetzt. Nach Abdampfen des Lösungsmittels wird am Kieselgel mit Chloroform chromatographiert.

Ausbeute: 104 mg (45% der Theorie)
Drehwert $[\alpha]_D^{20} = 42.46$ (Dioxan)

**Patentansprüche**

1. (+)- und (−)-Enantiomere von 5-Nitrodihydropyridinen der allgemeinen Formel I

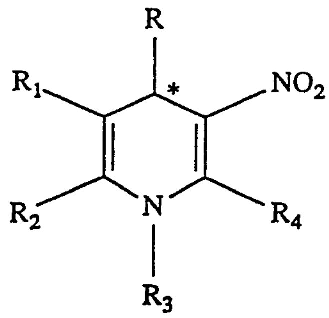

(I)

im welcher

R für Furyl, Thienyl, Pyridyl, Pyrimidyl, oder für Phenyl steht, welches gegebenenfalls ein- oder zweimal, gleich oder unterschiedlich substituiert ist durch Fluor, Chlor, Nitro, Cyano oder durch gegebenenfalls durch ein oder mehrere Fluor substituiertes Alkyl oder Alkoxy (jeweils 4 C-Atome), oder durch die Gruppe

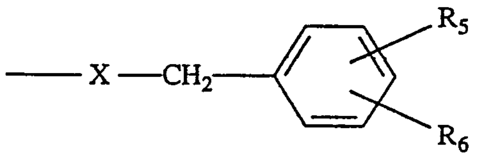

, wobei

X für Sauerstoff oder Schwefel steht, und

$R_5$ und $R_6$ gleich oder verschieden sind und für Wasserstoff, Nitro, Cyano oder für Alkyl, Alkyloxy, Alkylthio, Halogenalkyl, Halogenalkoxy (jeweils bis 2 C-Atome) steht und Halogen bevorzugt für ein oder mehrere Fluor steht,

$R_1$ für Wasserstoff oder für die Gruppe $CO_2R_8$ steht, wobei

$R_8$ für Wasserstoff oder für geradkettiges oder verzweigtes Alkyl (bis 6 C-Atome steht, das durch ein Sauerstoff- und/oder Schwefelatom in der Kette unterbrochen sein kann und das gegebenenfalls substituiert ist durch Phenyl, Nitro, ein oder mehrere Fluor, Chlor, Cyano, Benzyl-methylamino oder Pyridyl,

$R_2$, $R_4$ für Methyl oder Ethyl steht, das gegebenenfalls substituiert ist durch Hydroxy, ein oder mehrere Fluor, Phenyl oder Alkoxycarbonyl (bis 2 C-Atome),

$R_3$ für Wasserstoff oder für Methyl oder Ethyl steht, sowie deren Salze.

2. (−)-Enantiomere der allgemeinen Formel (I) in Anspruch 1.

3. Arzneimittel enthaltend als Wirkstoff (+)- bzw. (−)-Enantiomere der Formel (I) der Ansprüche 1 und 2.

4. Verfahren zur Herstellung von (+)- bzw. (−)-Enantiomeren der allgemeinen Formel I

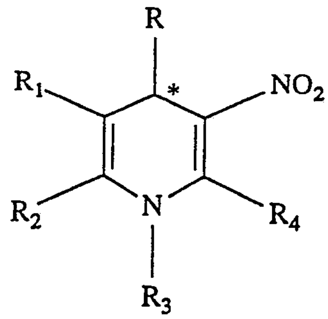

(I)

in welcher

R für Furyl, Thienyl, Pyridyl, Pyrimidyl, oder für Phenyl steht, der gegebenenfalls ein- oder zweimal, gleich oder unterschiedlich substituiert ist durch Fluor, Chlor, Nitro, Cyano oder durch gegebenenfalls durch ein oder mehrere Fluor substituiertes Alkyl oder Alkoxy (jeweils 4 C-Atome), oder durch die Gruppe

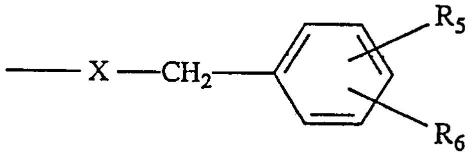

, wobei

X für Sauerstoff oder Schwefel steht, und

$R_5$ und $R_6$ gleich oder verschieden sind und für Wasserstoff, Nitro, Cyano oder für Alkyl, Alkyloxy, Alkylthio, Halogenalkyl, Halogenalkoxy (jeweils bis 2 C-Atome) steht und Halogen bevorzugt für ein oder mehrere Fluor steht,

$R_1$ für Wasserstoff oder für die Gruppe $CO_2R_8$ steht, wobei

$R_8$ für Wasserstoff oder für geradkettiges oder verzweigtes Alkyl (bis 6 C-Atome steht, das durch ein Sauerstoff- und/oder Schwefelatom in der Kette unterbrochen sein kann und das gegebenenfalls substituiert ist durch Phenyl, Nitro, ein oder mehrere Fluor, Chlor, Cyano, Benzyl-methylamino oder Pyridyl,

$R_2$, $R_4$ für Methyl oder Ethyl steht, das gegebenenfalls substituiert ist durch Hydroxy, ein oder mehrere Fluor, Phenyl oder Alkoxycarbonyl (bis 2 C-Atome),

$R_3$ für Wasserstoff oder für Methyl oder Ethyl steht, sowie deren Salze, dadurch gekennzeichnet, daß man die optisch aktiven Aminocrotonsäureester der allgemeinen Formel (II)

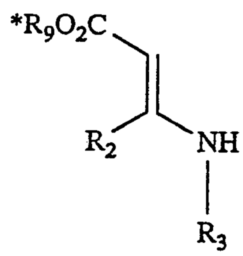

(II)

in welcher

$R_2$, $R_3$ die oben angegebene Bedeutung haben und

$^*R_9$ für einen chiral einheitlichen 2-Methoxy-2-phenyl-ethylrest steht,

mit Ylidenverbindungen der allgemeinen Formel II

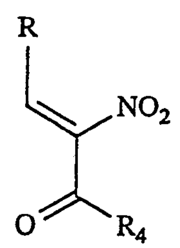

(III)

gegebenenfalls in Anwesenheit von Wasser oder inerten organischen Lösungsmitteln umsetzt, anschließend die hierbei anfallenden Diastereomeren nach üblichen Methoden trennt, und die so erhaltenen 1,4-Dihydropyridine mit chiraler Estergruppierung der allgemeinen Formel IV

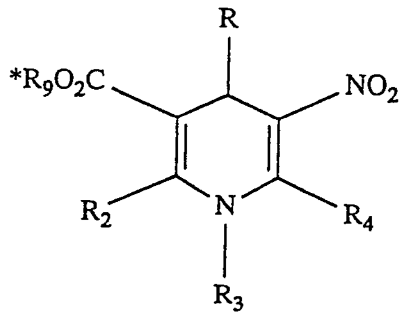

(IV)

in welcher

R, $R_1$, $R_2$, $R_3$, $R_4$ und $*R_9$ die oben angegebenen Bedeutung haben, nach üblichen Methoden entweder umestert, indem man den chiralen Rest $*R_9$ gegen einen achiralen Rest $R_8$ ($\neq$ Wasserstoff) austauscht, oder hydrolysiert zu Verbindungen mit $R_8$ = Wasserstoff, und diese anschließend gegebenenfalls wieder verestert oder decarboxyliert zu Verbindungen mit $R_1$ = H.

5. Verbindungen gemäß Anspruch 1 zur Verwendung bei der Bekämpfung von Erkrankungen.

6. Verwendung der Verbindungen gemäß Anspruch 1 zur Herstellung von Mitteln zur Prophylaxe und Therapie akuter und chronischer ischämischer Herzkrankheiten, zur Behandlung von cerebralen und peripheren Durchblutungsstörungen, zur Verbesserung der Herzkontraktilität, zur Senkung von Blutzucker, zur Abschwellung von Schleimhäuten und zur Beeinflussung des Salze- und Flüssigkeitshaushaltes.

7. Verfahren zur Herstellung von Arzneimitteln, dadurch gekennzeichnet, daß man Verbindungen gemäß Anspruch 1 mit üblichen Hilfs- und Trägerstoffen mischt.

8. Mischungen enthaltend (−)-Enantiomere bzw. (+)-Enantiomere gemäß Anspruch 1.

9. Mischungen enthaltend 0,01—1000 Gewichtsteile des (+)-Enantiomeren und 1 Gewichtsteil des (−)-Enantiomeren.

10. Verfahren zur Herstellung von Arzneimitteln enthaltend Mischungen gemäß Ansprüche 8—9, dadurch gekennzeichnet, daß man die Enantiomeren in inerten Lösungsmitteln auflöst, diese mischt, anschließend das Lösungsmittel entfernt und zusammen mit Hilfs- und Trägerstoffen in eine geeignete Applikationsform überführt.

**Revendications**

1. Enantiomères (+) et (−) de 5-nitrodihydropyridines de formule générale I

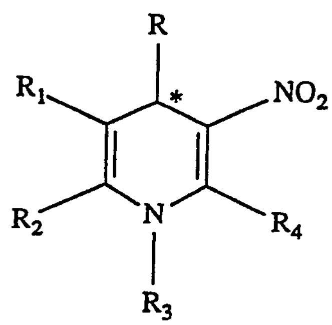

(I)

dans laquelle

R représente un groupe furyle, thiényle, pyridyle, pyrimidyle, ou représente un groupe phényle, éventuellement substitué une ou deux fois, de manière identique ou différente, par le fluor, le chlore, un groupe nitro, cyano ou par un groupe alkyle ou alcoxy (chaque fois 4 atomes de carbone) éventuellement substitué par un ou plusieurs atomes de fluor, ou par le groupe

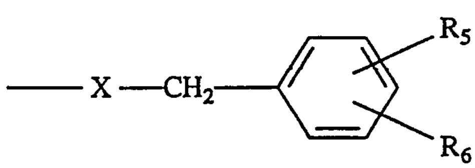

dans lequel

X représente l'oxygène ou le soufre, et

$R_5$ et $R_6$ sont identiques ou différents et représentent l'hydrogène, un groupe nitro, cyano ou un groupe alkyle, alcoxy, alkylthio, halogénoalkyle, halogénoalcoxy (chaque fois jusqu'à 2 atomes de carbone), l'halogène étant de préférence un ou plusieurs atomes de fluor,

$R_1$ représente l'hydrogène ou le groupe $CO_2R_8$, dans lequel

$R_8$ représente l'hydrogène ou un groupe alkyle linéaire un ramifié (jusqu'à 6 atomes de carbone) et pouvant étre interrompu dans la chaîne par un atome d'oxygène et/ou un atome de soufre et étant éventuellement substitué par un groupe phényle, nitro, un ou plusieurs fluor, chlore, cyano, benzyl-méthylamino ou pyridyle,

$R_2$, $R_4$ représentent un groupe méthyle ou éthyle, éventuellement substitué par hydroxy, un ou plusieurs atomes de fluor, un groupe phényle ou alcoxycarbonyle (jusqu'à 2 atomes de carbone),

$R_3$ représente l'hydrogène ou un groupe méthyle ou éthyle, ainsi que leurs sels.

2. Enantiomères (−) de formule générale (I) de la revendication 1.

3. Médicaments contenant comme substance active les énantiomères (+) ou (−) de formule (I) des revendications 1 et 2.

4. Procédé pour la fabrication des énantiomères (+) et (−) de formule générale I

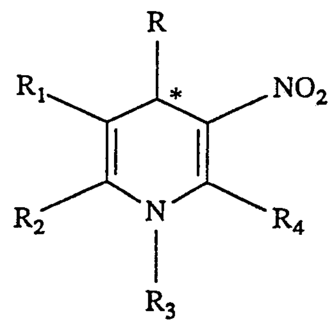

(I)

dans laquelle

R représente un groupe furyle, thiényle, pyridyle, pyrimidyle, ou représente un groupe phényle, éventuellement substitué une ou deux fois, de manière identique ou différente, par le fluor, le chlore, un groupe nitro, cyano ou par un groupe alkyle ou alcoxy (chaque fois 4 atomes de carbone) éventuellement substitué par un ou plusieurs atomes de fluor, ou par le groupe

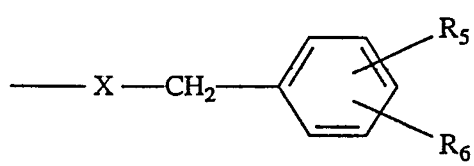

dans lequel

X représente l'oxygène ou le soufre, et

$R_5$ et $R_6$ sont identiques ou différents et représentent l'hydrogène, un groupe nitro, cyano ou un groupe alkyle, alcoxy, alkylthio, halogénoalkyle, halogénoalcoxy (chaque fois jusqu'à 2 atomes de carbone), et l'halogène est de préférence un ou plusieurs atomes de fluor,

$R_1$ représente l'hydrogène ou le groupe $CO_2R_8$, dans lequel

$R_8$ représente l'hydrogène ou un groupe alkyle linéaire un ramifié (jusqu'à 6 atomes de carbone) et pouvant étre interrompu dans la chaîne par un atome d'oxygène et/ou un atome de soufre et étant éventuellement substitué par un groupe phényle, nitro, un ou plusieurs fluor, chlore, cyano, benzyl-méthylamino ou pyridyle,

$R_2$, $R_4$ représentent un groupe méthyle ou éthyle, éventuellement substitué par hydroxy, un ou plusieurs atomes de fluor, un groupe phényle ou alcoxycarbonyle (jusqu'à 2 atomes de carbone),

$R_3$ représente l'hydrogène ou un groupe méthyle ou éthyle, ainsi que leurs sels, caractérisé en ce que l'on fait réagir, éventuellement en présence d'eau ou de solvants organiques inertes, les esters optiquement actifs de l'acide aminocrotonique de formule générale (II)

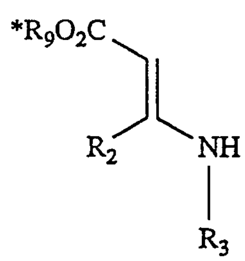

(II)

dans laquelle

$R_2$, $R_3$ ont les significations décrites ci-dessus et

$^*R_9$ représente un reste à chiralité unique 2-méthoxy-2-phényl-éthyle, avec des composés d'ylidène de formule générale III

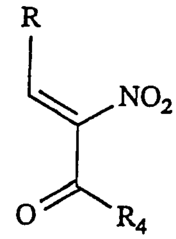

(III)

puis qu'on sépare les diastéréomères produits par des méthodes usuelles, et qu'on transestérifie par des méthodes usuelles les 1,4-dihydropyridines à groupement ester chiral ainsi obtenues, répondant à la formule générale IV

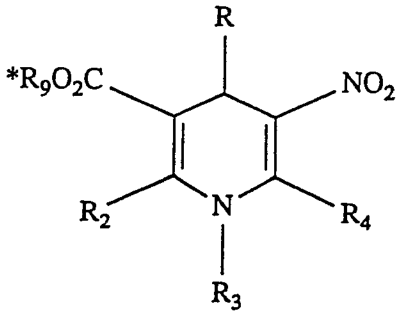

(IV)

dans laquelle

R, $R_1$, $R_2$, $R_3$, $R_4$ et $^*R_9$ ont les significations décrites ci-dessus, en échangeant les reste chiral $^*R_9$ contre un reste achiral $R_8$ ($\neq$ hydrogène), ou qu'on les hydrolyse en composés avec $R_8$ = hydrogène, et qu'éventuellement ensuite on estérifie à nouveau ou on décarboxyle ces derniers en composés avec $R_1$ = H.

5. Composés selon la revendication 1 pour utilisation dans la lutte contre les maladies.

6. Utilisation des composés selon la revendication 1 pour la fabrication de moyens pour la prophylaxie et la thérapie d'affections cardiaques ischémiques aiguës et chroniques, pour le traitement de troubles cérébraux et périphériques d'irrigation sanguine, pour l'amélioration de la contractilité cardiaque, pour abaisser la teneur en sucre dans la sang, pour la décongestion des muqueuses et pour influencer l'économie des sels et des liquides.

7. Procédé pour la fabrication de médicaments, caractérisé en ce que l'on mélange les composés selon la revendication 1 avec des adjuvants et vecteurs usuels.

8. Mélanges contenant des énantiomères (−) et des énantiomères (+) selon la revendication 1.

9. Mélanges contenant 0,01—1000 parties en poids d'énantiomère (+) et une partie en poids d'énantiomère (−).

10. Procédé pour la fabrication de médicaments contenant des mélanges selon les revendications 8—9, caractérisé en ce que l'on dissout les énantiomères dans des solvants inertes, qu'on les mélange, puis qu'on élimine le solvant et qu'on passe à une forme d'application appropriée avec des adjuvants et des vecteurs.

**Claims**

1. (+)- and (−)-enantiomers of 5-nitrodihydropyridines of the general formula I

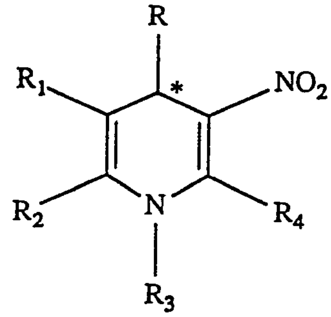

(I)

in which

R represents furyl, thienyl, pyridyl or pyrimidyl, or represents phenyl which is optionally

monosubstituted, or disubstituted, identically or differently, by fluorine, chlorine, nitro or cyano or by alkyl or alkoxy (4 C atoms in each case) which is optionally substituted by one or more fluorine atoms, or by the group

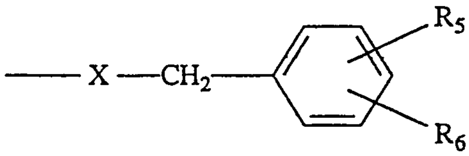

wherein

X represents oxygen or sulphur and

$R_5$ and $R_6$ are identical or different and represent hydrogen, nitro or cyano or alkyl, alkoxy, alkylthio, halogenoalkyl and halogenoalkoxy (in each case up to 2 C fluorine atoms),

$R_1$ represents hydrogen or the group $CO_2R_8$ wherein

$R_8$ represents hydrogen or linear or branched alkyl (up to 6 C atoms) which can be interrupted by an oxygen and/or sulphur atom in the chain and which is optionally substituted by phenyl, nitro or one or more fluorine, chlorine, cyano, benzylmethylamino or pyridyl groups, $R_2$ and $R_4$ represent methyl or ethyl which is optionally substituted by hydroxyl or one or more fluorine, phenyl or alkoxycarbonyl (up to 2 C atoms) groups and

$R_3$ represents hydrogen or methyl or ethyl, and salts thereof.

2. (−)-Enantiomers of the general formula (I) in Claim 1.

3. Medicaments containing (+)- and/or (−)-enantiomers of the formula (I) of Claims 1 and 2 as the active compound.

4. Process for the preparation of (+)- and/or (−)-enantiomers of the general formula I

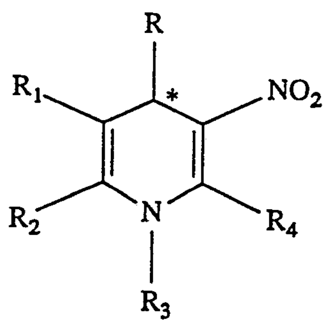

(I)

in which

R represents furyl, thienyl, pyridyl or pyrimidyl, or represents phenyl which is optionally monosubstituted, or disubstituted, identically or differently, by fluorine, chlorine, nitro or cyano or by alkyl or alkoxy (4 C atoms in each case) which is optionally substituted by one or more fluorine atoms, or by the group

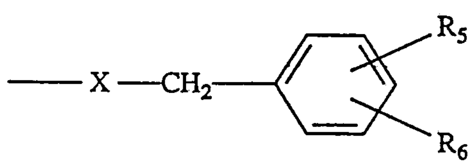

wherein

X represents oxygen or sulphur and

$R_5$ and $R_6$ are identical or different and represent hydrogen, nitro or cyano or alkyl, alkoxy, alkylthio, halogenoalkyl and halogenoalkoxy (in each case up to 2 C atoms), and halogen preferably represents one or more fluorine atoms,

$R_1$ represents hydrogen or the group $CO_2R_8$ wherein

$R_8$ represents hydrogen or linear or branched alkyl (up to 6 C atoms) which can be interrupted by an oxygen and/or sulphur atom in the chain and which is optionally substituted by phenyl, nitro or one or more fluorine, chlorine, cyano, benzylmethylamino or pyridyl groups,

$R_2$ and $R_4$ represent methyl or ethyl which is optionally substituted by hydroxyl or one or more fluorine, phenyl or alkoxycarbonyl (up to 2 C atoms) groups and

$R_3$ represents hydrogen or methyl or ethyl and salts thereof, characterized in that the optically active aminocrotonic acid esters of the general formula (II)

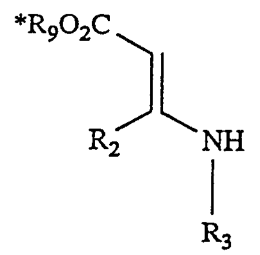

(II)

in which

$R_2$ and $R_3$ have the meaning indicated above and

$*R_9$ represents a chirally uniform 2-methoxy-2-phenylethyl radical, are reacted with ylidene compounds of the general formula III

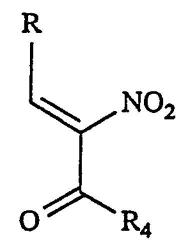

(III)

if desired in the presence of water or inert organic solvents, the diastereomers thus produced are then separated by customary methods and the resulting 1,4-dihydropyridines having a chiral ester grouping of the general formula IV

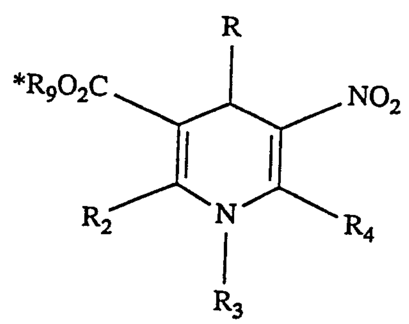

(IV)

in which

R, $R_1$, $R_2$, $R_3$, $R_4$ and $*R_9$ have the meaning indicated above, are either transesterified in accordance with customary methods by replacing the chiral radical $*R_9$ by a non-chiral radical $R_8$ ($\neq$ hydrogen), or are hydrolysed to give compounds in which $R_8$ = hydrogen, and these compounds are then, if desired, re-esterified or decarboxylated to give compounds in which $R_1$ = H.

5. Compounds according to Claim 1 for use in the combating of diseases.

6. The use of the compounds according to Claim 1 for the preparation of agents for the prophylaxis and therapy of acute and chronic ischaemic cardiac disorders, for the treatment of cerebral and peripheral circulatory disturbances, for improving the contractility of the heart, for reducing blood sugar levels, for causing detumescence of mucous membranes and for influencing the salt and liquid balance.

7. Process for the preparation of medicaments, characterized in that compounds according to Claim 1 are mixed with customary auxiliaries and excipients.

8. Mixtures containing (−)-enantiomers and (+)-enantiomers according to Claim 1.

9. Mixtures containing 0.01—1,000 parts by weight of the (+)-enantiomer and 1 part by weight of the (−)-enantiomer.

10. Process for the preparation of medicaments containing mixtures according to Claims 8—9, characterized in that the enantiomers are dissolved in inert solvents, these solutions are mixed and the solvent is then removed and the product is converted, together with auxiliaries and excipients, into a suitable application form.